# EUROPÄISCHE PATENTANMELDUNG

(11) **EP 1 493 389 A1**
(43) Veröffentlichungstag der Anmeldung: **05.01.2005**
(21) Anmeldenummer: 04013523.8
(22) Anmeldetag: 08.06.2004
(51) Int. Cl.: A61B 6/12

(54) **Verfahren und Einrichtung zum Erzeugen eines Röntgenbildes aus der Fokusregion eines Lithotripters**

(30) Priorität: 01.07.2003 DE 10329299
(71) Anmelder: SIEMENS AKTIENGESELLSCHAFT, 80333 München (DE)
(72) Erfinder: Kaltschmidt, Rainer, 90542 Eckental/Brand (DE)

(57) **Zusammenfassung**

Bei einem Verfahren und einer Einrichtung zum Erzeugen eines Röntgenbildes (B) aus der Fokusregion (8) eines Lithotripters (1) mit Hilfe von Röntgenstrahlen, die die Fokusregion (8) und Geräteteile des Lithotripters (1) durchsetzen, wird zur Steuerung der Dosisleistung der Röntgenstrahlen ausschließlich ein Bildbereich (36) des von den Röntgenstrahlen erfassten Bildfeldes (32) ausgewertet, der außerhalb eines durch diese Geräteteile abgeschatteten Gebietes (30) liegt.

## Beschreibung

Die Erfindung bezieht sich auf ein Verfahren zum Erzeugen eines Röntgenbildes aus der Fokusregion eines Lithotripters und auf eine Einrichtung zum Durchführen des Verfahrens.

Bei der Lithotripsie handelt es sich um eine therapeutische Methode, ein im Körper eines Lebewesens befindliches Konkrement ohne operativen Eingriff mit Hilfe einer fokussierten Ultraschall-Stoßwelle zu zerstören, die von einem Stoßwellengenerator erzeugt wird. Um Schäden im das Konkrement umgebenenden Gewebe weitgehend zu vermeiden, ist es einerseits erforderlich, den Fokus des Lithotripters exakt im Konkrement zu positionieren. Andererseits ist es auch notwendig, den Fortschritt bei der Zerstörung des Konkrements zu visualisieren, um die Anzahl der erforderlichen Stoßwellen-Impulse auf das therapeutisch notwendige Minimum zu begrenzen.

Zum Erzeugen eines Röntgenbildes aus der Umgebung der Fokusregion eines Lithotripters ist es beispielsweise aus der DE 197 46 956 A1 bekannt, ein Röntgengerät mit einem Lithotripter derart zu koppeln, dass die vom Röntgengerät erzeugten Röntgenstrahlen die Fokusregion des Lithrotripters durchsetzen. Bei dieser bekannten Einrichtung handelt es sich um eine so genannte "off-line"- oder "out-line"-Anordnung, bei der die Mittenachse der Röntgenstrahlung gegenüber der Mittenachse der vom Stoßwellengenerator erzeugten fokussierten Stoßwelle geneigt angeordnet ist und sich die Fokusregion annähernd im Schnittpunkt dieser Mittenachsen befindet.

Alternativ oder ergänzend hierzu ist es im Stand der Technik auch bekannt, ein Röntgenbild aus der Fokusregion mit Hilfe einer so genannten "in-line"-Anordnung zu erzeugen, bei der die Mittenachse der zur Bildwiedergabe erzeugten Röntgenstrahlen mit der Mittenachse der fokussierten Stoßwelle zusammenfällt.

Während einer solchen Lithotripsie-Behandlung ist es sowohl zu exakten Positionierung des Fokus der Stoßwelle als auch zur Visualisierung der fortschreitenden Zertrümmerung des bzw. der Konkremente notwendig, eine Vielzahl von Röntgenbildern aufzunehmen. Trotz des Einsatzes von Röntgenbildverstärkern und der dadurch möglichen Verwendung von Röntgenquellen mit relativ geringer Dosisleistung ist es nach wie vor wünschenswert, die Dosisbelastung des Patienten weiter zu verringern.

Der Erfindung liegt nun die Aufgabe zugrunde, ein Verfahren zum Erzeugen eines Röntgenbildes aus der Fokusregion eines Stoßwellengenerators anzugeben, mit dem eine Verringerung der Dosisbelastung des Patienten ermöglicht ist. Außerdem liegt der Erfindung die Aufgabe zugrunde, eine nach diesem Verfahren arbeitende Einrichtung anzugeben.

Die erstgenannte Aufgabe wird gemäß der Erfindung gelöst mit einem Verfahren mit den Merkmalen des Patentanspruchs 1. Da zur Steuerung der Dosisleistung der Röntgenstrahlung ausschließlich ein Bildbereich des von der Röntgenstrahlung erfassten Bildfeldes ausgewertet wird, der außerhalb eines von Geräteteilen des Lithotripters abgeschatteten Gebietes liegt, ist es möglich, die Bildqualität und damit die Strahlungsdosis gezielt für diesen Bildbereich zu optimieren.

Die Erfindung geht dabei von der Erkenntnis aus, dass bei Röntgenbildern aus der Fokusregion eines Lithotripters abgeschattete und diagnostisch nicht nutzbare Bildbereiche existieren, die nicht vermeidbar sind. Dies ist bei einer "outline"-Anordnung der von einem Teil der Röntgenstrahlung durchsetzte und teilweise abgebildete Koppelbalg des Stoßwellengenerators, der eine Schwächung der Röntgenstrahlung bewirkt. Bei einer "in-line"-Anordnung ist es einerseits die Umgebung einer für die Röntgenstrahlung verwendeten Lochblende und andererseits ebenfalls die von der Röntgenstrahlung zusätzlich zu durchquerende Wasservorlaufstrecke im Koppelbalg. Diese verringern die auf das gesamte Bildfeld bezogene Helligkeit des Röntgenbildes und bewirken, dass die im Stand der Technik verwendete automatische Dosisleistungssteuerung sowohl die Dosisleistung als auch die Betriebsspannung der Röntgenröhre erhöht. Dies führt neben einer Erhöhung der Dosisbelastung aufgrund der geringeren Primärabsorption bei höherenergetischen Röntgenquanten zusätzlich zu einer Verschlechterung des Bildkontrastes und damit der Erkennbarkeit des Konkrements.

Die Erfindung beruht dabei auf der Idee, zur Aussteuerung der Röntgenanlage nur diejenigen Teile des gesamten Bildfeldes, zur Belichtungssteuerung zu verwerten, die diagnostisch nutzbar und von vorrangigem Interesse sind und vorzugsweise außerhalb eines abgeschatteten Gebietes liegen und die unmittelbare Umgebung des Fökus des Lithotripters umfassen. Es wird demzufolge gemessen an der mittleren Helligkeit des gesamten Bildfeldes gezielt eine Unterbelichtung vorgenommen, die jedoch ausreichend ist, um den primär diagnostisch interessierenden Anteil des gesamten Bildfeldes in guter Bildqualität darzustellen. Durch diese Maßnahme wird demzufolge die Strahlenbelastung des Patienten deutlich reduziert, ohne dass es zu einer störenden Verschlechterung der Wiedergabequalität in diesen diagnostisch interessierenden Bereichen kommt.

In einer vorteilhaften Ausgestaltung des Verfahrens wird das abgeschattete Gebiet des Bildfeldes mit einem digitalen Bildbearbeitungsverfahren aufgehellt. Dadurch ist es möglich, trotz der Unterbelichtung auch dieses Gebiet noch diagnostisch, insbesondere für die Steinortung, zu verwerten.

Weitere vorteilhafte Ausgestaltungen des Verfahrens ergeben sich gemäß den weiteren Unteransprüchen.

Die zweitgenannte Aufgabe wird gemäß der Erfindung gelöst mit einer Einrichtung mit den Merkmalen des Patentanspruches 10, dessen Vorteile sich sinngemäß aus den Vorteilen des entsprechenden Verfahrensanspruches 1 ergeben.

Weitere vorteilhafte Ausgestaltungen der Einrichtung sind in den zugehörigen Unteransprüchen wiedergegeben, deren Vorteile sich ebenfalls sinngemäß aus den Vorteilen der ihnen jeweils entsprechenden Verfahrensansprüche ergeben.

Zur weiteren Erläuterung der Erfindung wird auf die Zeichnung verwiesen. Es zeigen:
- Fig. 1: eine Einrichtung gemäß der Erfindung in einer schematischen Prinzipdarstellung,
- Fig. 2 und 3: jeweils eine vorteilhafte Auswahl des zur Auswertung herangezogenen Bildbereiches bei einer "in-line"-Anordnung bzw. des zur Auswertung herangezogenen Bildbereiches bei einer "out-line"-Anordnung.

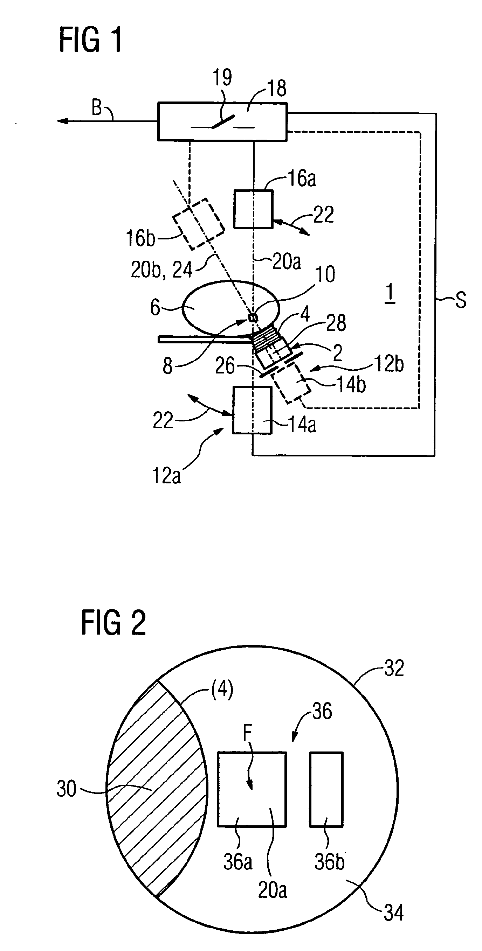

Eine in Fig. 1 schematisch dargestellte Einrichtung gemäß der Erfindung umfasst einen Lithotripter 1, dessen Stoßwellengenerator 2 über einen mit Wasser gefüllten Koppelbalg 4 auf die Oberfläche des Körpers 6 eines Lebenwesens aufgesetzt ist. Der Stoßwellengenerator 2 erzeugt eine Ultraschall-Stoßwelle, die in einer Fokusregion 8 fokussiert ist. Bei korrekter Positionierung des Stoßwellengenerators 2 befindet sich ein zu zerstörendes Konkrement 10 in dieser Fokusregion 8.

Dem Lithotripter 1 ist eine Röntgeneinrichtung 12a zugeordnet, die eine Röntgenquelle 14a sowie einen Röntgenempfänger 16a umfasst. Aus dem vom Röntgenempfänger 16a empfangenen Röntgenbild wird nach dessen Digitalisierung und Auswertung in einer Steuereinrichtung 18 ein Steuersignal S zur erfindungsgemäßen kontrast- und dosisoptimierten Steuerung der Röntgenquelle 14a erzeugt. Ein auf diese Weise erzeugtes optimiertes Röntgenbild B wird ausgegeben und beispielsweise in einem Monitor wiedergegeben. Mit Hilfe eines in der Figur symbolisch veranschaulichten Wahlschalters 19 kann der Anwender entscheiden, ob eine kontrast- und dosisoptimierte Steuerung der Röntgenquelle 14a gemäß der Erfindung erfolgen soll oder ob kurzzeitig eine konventionelle Steuerung gewünscht ist, um auch die abgeschatteten Bereiche diagnostisch nutzen zu können.

Die Mittenachse 20a der von der Röntgenquelle 14a emittierten Röntgenstrahlung, d.h. die Verbindungslinie zwischen Röntgenquelle 14a und Röntgenempfänger 16a, ist derart positioniert, dass diese die Fokusregion 8 des Lithotripters 2 schneidet. Mit anderen Worten: Die von der Röntgenquelle 14a emittierten Röntgenstrahlen durchsetzen die Fokusregion 8 des Lithotripters. Röntgenquelle 14a und Röntgenempfänger 16a können dabei auf einem so genannten C-Bogen, in der Figur symbolisch durch Doppelpfeile 22 veranschaulicht, angeordnet sein und um den Schnittpunkt zwischen Mittenachse 20a der Röntgenstrahlung und Mittenachse 24 der vom Stoßwellengenerator 2 emittierten Ultraschall-Stoßwelle gemeinsam geschwenkt werden.

Bei der in der Figur dargestellten Röntgeneinrichtung 12a handelt es sich um eine so genannte "out-line"-Anordnung, bei der Mittenachse 20a der Röntgenstrahlung und Mittenachse 24 der Ultraschall-Stoßwelle zueinander geneigt sind, d.h. nicht zusammenfallen.

In Figur 1 ist gestrichelt alternativ oder ergänzend zur Röntgeneinrichtung 12a eine Röntgeneinrichtung 12b in einer so genannten "in-line"-Anordnung eingezeichnet, bei der Mittenachse 20b der Röntgenstrahlung und Mittenachse 24 der Ultraschall-Stoßwelle zusammenfallen. Eine in der Figur schematisch dargestellte Blende 26 vor der Röntgenquelle 16b begrenzt die transmittierte Röntgenstrahlung auf einen zentralen Bereich um die Mittenachse 20b bzw. 24. Bei einer "inline"-Anordnung ist es, wie in der Figur dargestellt, üblich, die Röntgenquelle 14b hinter dem mit einer Durchtrittsöffnung 28 versehenen Stoßwellengenerator 2 anzuordnen. Dies hat den Vorteil, dass die auch im Bereich der Mittenachse 24 aufgrund der Wasservorlaufstrecke unvermeidliche zusätzliche Absorption der Röntgenstrahlung und die deshalb erforderliche höhere Dosisleistung nicht zu einer erhöhten Strahlenexposition des Patienten führt, da die Absorption stattfindet, bevor die Röntgenstrahlung in den Körper 6 eintritt. Dennoch würde auch in diesem Fall eine Aussteuerung der Röntgenanlage nach dem Stand der Technik zu einer Überbelichtung des zentralen Bereiches und aufgrund der höherenergetischen Röntgenquanten zu einer unerwünschten Kontrastminderung führen. Grundsätzlich können jedoch auch die Positionen von Röntgenquelle 14b und Röntgenempfänger 16b vertauscht sein, so dass der Röntgenempfänger 16b hinter dem Stoßwellengenerator 2 angeordnet ist. Auch in diesem Fall ist die Blende 26 unmittelbar vor der Röntgenquelle 14b angeordnet.

Gemäß Figur 2 hat nun das vom Röntgenempfänger 16a empfangene Röntgenbild eine Struktur, die einem "¾-Mond" ähnlich erscheint. In der Figur ist durch eine Schraffur hervorgehoben zu erkennen, dass ein Teil 30 des vom Röntgenempfänger 16a (Figur 1) erfassten Bildfeldes 32, d.h. des Austastkreises des Röntgenbildverstärkers, abgeschattet ist. Diese Abschattung entsteht dadurch, dass Geräteteile des Lithotripters 1, im Wesentlichen der Koppelbalg 4, in den Strahlengang der Röntgenstrahlung hineinragen und zu einer verstärkten Absorption der Röntgenstrahlung in der Balgwand und im Koppelmedium führt. Um nun eine Überbelichtung des außerhalb dieses abgeschatteten Teils 30 liegenden Gebietes 34 zu vermeiden, werden zur Steuerung der Röntgenquelle 14a (Figur 1) nur die Bilddaten verwendet, die aus einem Bildbereich 36 stammen, der im normal belichteten Teil 34 liegt. Im Ausführungsbeispiel besteht der Bildbereich 36 aus zwei rechteckigen, räumlich voneinander getrennten Teilbereichen 36a,36b, von denen der eine die Mittenachse 20a und damit unmittelbare Umgebung des Fokus F der Ultraschall-Stoßwelle umfasst und der andere außerhalb dieser unmittelbaren Umgebung neben dem Fokus F aber noch innerhalb des normal belichteten Teils 34 angeordnet ist. Die integralen Helligkeiten beider Teilbereiche 36a,b werden zur Steuerung der Intensität der Röntgenstrahlung und damit der applizierten Dosis herangezogen, wobei jedoch die aus dem Teilbereich 36a ermittelten Daten eine höhere Gewichtung erfahren als die Daten, die aus dem Teilbereich 36b ermittelt worden sind. Es folgt also nicht wie im Stand der Technik eine Aussteuerung der Röntgeneinrichtung aufgrund der Helligkeit des gesamten erfassten Bildfeldes 32 sondern nur aufgrund der Helligkeit eines oder mehrerer vorbestimmter Bildbereiche 36a,b, die im normal belichteten Teil des Bildfeldes liegen. Dies hat zur Folge, dass der bereits bei einer Steuerung der Röntgenanlage gemäß dem Stand der Technik unterbelichtete abgeschattete Teil 30 zusätzlich abgeschwächt wird. Um trotz dieser erfindungsgemäßen Unterbelichtung den abgeschatteten Teil 30 auch ohne Erhöhung der Dosisleistung noch diagnostisch, beispielsweise zur Erleichterung der Orientierung, nutzen zu können, werden die im abgeschatteten Teil 30 vorliegenden Daten in einer digitalen Bildbearbeitungseinrichtung unabhängig vom normal belichteten Teil 34 zur Kontrastoptimierung nachbearbeitet.

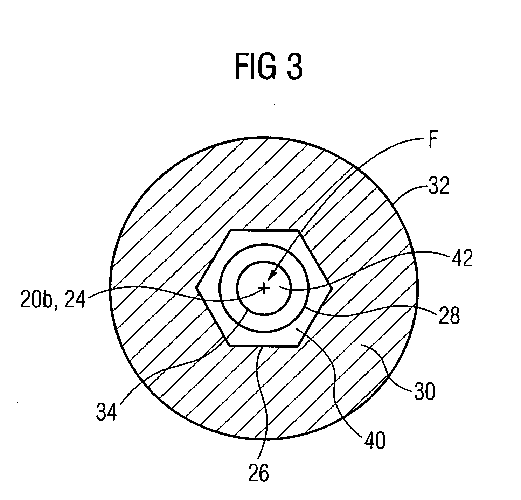

Fig. 3 zeigt nun die Abbildungsverhältnisse bei einer "inline"-Anordnung, bei der der Röntgenempfänger abweichend von der gestrichelt in Fig. 1 dargestellten Positionierung im Bereich der Stoßwellenquelle angeordnet ist. Auch in diesem Fall ist ein großer Teil 30 des gesamten Bildfeldes 32 durch die Blende 26 abgeschattet. Zwischen dem Bild der Durchtrittsöffnung 28 und der Blende 26 befindet sich nun ein ebenfalls nur schwach belichteter Teil 40 des Bildfeldes 32. In der Mitte des Bildfeldes 32, und begrenzt durch die Durchtrittsöffnung 28 befindet sich der normal belichtete Teil 34. Zur Steuerung der Dosisleistung der Röntgenquelle 14b (Figur 1) wird nun ein zentraler Bildbereich 42 verwendet, der die unmittelbare Umgebung um den Fokus F und die Mittenachse 20b der Röntgenstrahlung sowie die Mittenachse 24 der Ultraschall-Stoßwelle umfasst. Auf diese Weise ist sichergestellt, dass nur der diagnostisch relevante Bereich innerhalb der Fokusregion korrekt belichtet wird, während in den übrigen Bildzonen eine ausgeprägte Unterbelichtung bewusst in Kauf genommen wird.

## Patentansprüche

1. Verfahren zum Erzeugen eines Röntgenbildes (B) aus der Fokusregion (8) eines Lithotripters (1) mit Hilfe von Röntgenstrahlen, die die Fokusregion (8) und Geräteteile des Lithotripters (1) durchsetzen, wobei zur Steuerung der Dosisleistung der Röntgenstrahlen ausschließlich ein Bildbereich (36;42) des von den Röntgenstrahlen erfassten Bildfeldes (32) ausgewertet wird, der außerhalb eines durch diese Geräteteile abgeschatteten Gebietes (30) liegt.

2. Verfahren nach Anspruch 1, bei dem die integrale Helligkeit des Bildbereichs (36;42) ausgewertet und zur Steuerung der Dosisleistung herangezogen wird.

3. Verfahren nach Anspruch 1 oder 2, bei dem der Bildbereich (36;42) außerhalb eines abgeschatteten Gebietes (30) angeordnet ist und die unmittelbare Umgebung des Fokus (F) des Lithotripters (1) umfasst.

4. Verfahren nach einem der vorhergehenden Ansprüche, bei dem das abgeschattete Gebiet (30) des Bildfeldes (32) mit einem digitalen Bildbearbeitungsverfahren aufgehellt wird.

5. Verfahren nach einem der vorhergehenden Ansprüche, bei dem Röntgenstrahlen und Stoßwellen eine gemeinsame Mittenachse (20b, 24) aufweisen und im Strahlengang eine Blende (26) angeordnet ist, und bei dem der Bildbereich (42) in der Mitte des von den Röntgenstrahlen erzeugten Röntgenbildes der Blende (26) angeordnet wird.

6. Verfahren nach Anspruch 5, bei dem der Bildbereich (42) kreisscheibenförmig ist.

7. Verfahren nach einem der Ansprüche 1 bis 4, bei dem Röntgenstrahlen und Stoßwellen zueinander geneigte Mittenachsen (20a, 24) aufweisen.

8. Verfahren nach Anspruch 7, bei dem der Bildbereich (36) wenigstens zwei voneinander räumlich getrennte Teilbereiche (36a, 36b) außerhalb des abgeschatteten Gebietes (30) umfasst.

9. Verfahren nach Anspruch 8, bei dem die Bildeigenschaften der Teilbereiche (36a, 36b) bei der Auswertung unterschiedlich gewertet werden.

10. Einrichtung zur Durchführung des Verfahrens nach einem der vorhergehenden Ansprüche, mit einem Lithotripter (1) zur Zertrümmerung eines Konkrements (10) im Körper (6) eines Lebewesens mit Hilfe fokussierter Stoßwellen und mit einer Röntgeneinrichtung (12a, 12b) zum Erzeugen eines Röntgenbildes (B) aus der Fokusregion (8) des Lithotripters (1) mit Hilfe von Röntgenstrahlen, die diese Fokusregion (8) und Geräteteile des Lithotripters (1) durchsetzen, wobei die Röntgeneinrichtung (12a, 12b) eine Steuereinrichtung (18) zur Steuerung der Dosisleistung der Röntgenstrahlen durch ausschließliche Auswertung eines Bildbereiches (36;42) des von den Röntgenstrahlen erfassten Bildfeldes (32) umfasst.

11. Einrichtung nach Anspruch 10, bei der die Steuerung der Dosisleistung nach einem Verfahren nach einem der Ansprüche 1 bis 9 abschaltbar ist.

12. Einrichtung nach Anspruch 10 oder 11, bei der die Steuereinrichtung (18) eine Einrichtung zum Auswerten der integralen Helligkeit des Bildbereiches (36;42) für die Steuerung der Dosisleistung umfasst.

13. Einrichtung nach einem der Ansprüche 10 bis 12, bei der der Bildbereich (36;42) außerhalb eines abgeschatteten Gebietes (30) angeordnet ist und die unmittelbare Umgebung des Fokus (F) des Lithotripters (1) umfasst.

14. Einrichtung nach einem der Ansprüche 10 bis 13, bei der das abgeschattete Gebiet (30) des Bildfeldes (32) mit einem digitalen Bildbearbeitungsverfahren aufgehellt wird.

15. Einrichtung nach einem der Ansprüche 10 bis 14, bei der Röntgenstrahlen und Stoßwellen eine gemeinsame Mittenachse (20b, 24) aufweisen und im Strahlengang eine Blende (26) angeordnet ist, und bei dem der Bildbereich (42) in der Mitte des von den Röntgenstrahlen erzeugten Röntgenbildes der Blende (26) angeordnet ist.

16. Einrichtung nach Anspruch 15, bei der der Bildbereich (42) kreisscheibenförmig ist.

17. Einrichtung nach einem der Ansprüche 10 bis 14, bei der Röntgenstrahlen und Stoßwellen zueinander geneigte Mittenachsen (20a, 24) aufweisen.

18. Einrichtung nach Anspruch 17, bei der der Bildbereich (36) wenigstens zwei voneinander räumlich getrennte Teilbereiche (36a, 36b) umfasst.

19. Einrichtung nach Anspruch 18, bei der die Bildeigenschaften der Teilbereiche (36a, 36b) bei der Auswertung unterschiedlich gewertet sind.
